# EUROPÄISCHE PATENTANMELDUNG

(11) **EP 1 982 758 A1**
(43) Veröffentlichungstag der Anmeldung: **22.10.2008**
(21) Anmeldenummer: 07106570.0
(22) Anmeldetag: 20.04.2007
(51) Int. Cl.: B01J 13/02, C08B 37/00, C12N 11/04

(54) **Verfahren zur Herstellung sulfatierter Polysaccharide**

(71) Anmelder: Euroferm Gesellschaft für Fermentation und Messtechnik mbH, 91052 Erlangen (DE)
(72) Erfinder: Fischer, Peter, Dr.-Ing., 91090 Effeltrich (DE); Fischbach, Matthias, Dr. rer. nat., 37073 Göttingen (DE); Geppert, Eric, Dipl.-Ing., 90766 Fürth (DE)
(74) Vertreter: Fitzner, Uwe

(57) **Zusammenfassung**

Die vorliegende Erfindung betrifft ein Verfahren Verfahren zur biotechnologischen Herstellung von sulfatierten Polysacchariden mittels tierischen Zellen, wobei die Zellen in immobilisierter Form eingesetzt werden.

## Beschreibung

Die Erfindung betrifft ein Verfahren zur biotechnologischen Herstellung von sulfatierten Polysacchariden, insbesondere Heparin.

Heparin ist ein bekanntes einwertiges oder zweiwertiges Salz der instabilen Herapinsäure. Es ist ein Polymerisat aus einer Disaccharideinheit in Form der folgenden Struktureinheit:

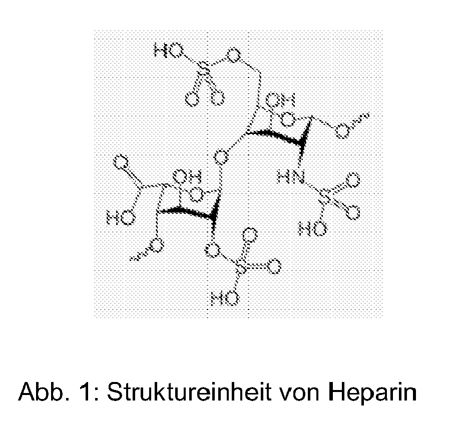

Herparine sind therapeutisch wirksame Polysaccharide zur Blutgerinnungshemmung. Bereits mit einer Kettenlänge von 5 Monosacchariden sind sie wirskam.

Die Antigerinnungseigenschaften von Heparin sind seit vielen Jahren bekannt. Heparin liegt in unterschiedlichen Formen in zahlreichen Geweben und Zellen vor. Beispielsweise sind in der Haut und in den Lungen von unterschiedlichen Tierarten Heparine in hochmolekularer Form vorhanden.

Die Herstellung von Heparin erfolgt fast ausschließlich durch Gewinnung aus tierischen Geweben, insbesondere Schweinedarmmukosa. Über verschiedene Degradationsschritte wird ein Gemisch aus unterschiedlich langen Mukopolysacchariden erhalten. Aus 1 Liter dieser Mukosa kann man nur etwa 150 mg unfranktioniertes Standardheparin isolieren. Daher müssen Tausende von Tonnen Schweinedärmen verarbeitet werden, um den weltweiten Bedarf an Heparin zu decken.

Verfahren zur Extraktion von Heparin aus Schlachtabfällen sind unter anderem in der DE 3010972, PCT/EP98/04939 und PCT/FR02/03617 beschrieben.

Sämtliche bekannte Verfahren weisen erhebliche Nachteile auf. Zum einen muss wegen des hohen Wassergehaltes der Organe mit einem beträchtlichen Heparinverlust gerechnet werden. Ferner vermehren sich während der Lagerung und der Autolyse die das Heparin schädigenden Mikroorganismen, was eine Verminderung der Ausbeute zur Folge hat. Eines der Hauptprobleme ergibt sich aus dem Sammeln der Organe, da durch die Schwankungen in der Zusammensetzung auch die Qualität des erhaltenen Produktes beeinflusst wird.

Das Wesen der zur Herstellung eingesetzten Extraktionsverfahren besteht darin, dass der Wirkstoffgehalt der heparinhaltigen Organe nur mittels Chemikalien aufgeschlossen und als Heparin oder Heparin/Eiweiß-Komplex in Lösung gebracht wird. Derartige Verfahren sind beispielsweise aus der HU 158776, HU 149339, GB 992201, US-PS 2,623,001, US 3,058,884 und US 3,262,854 bekannt. Auch bei diesen Verfahren verursachen die Zusammensetzungsschwankungen Unzulänglichkeiten und Unannehmlichkeiten, wie schwankende Ausgangszusammensetzungen und die mangelnde Reproduzierbarkeit der folgenden Aufarbeitungsstufen.

Bei einem weiteren bekannten Extraktionsverfahren werden die heparinhaltigen Organe zwecks Verminderung der Heparinverluste und Vermeidung der bei der Vorautolyse auftretenden Unsicherheiten nicht nur Chemikalien behandelt, sondern auch noch einer bis zur völligen oder fast völligen Auflösung führenden langwierigen und intensiven Proteolyse unterworfen, indem Enzyme, wie Tripsin, Pankreasextrakt, Papain bzw. Elastase in das System eingebracht werden. Derartige Verfahren sind z.B. aus der HU 147323, US 2,587,914, US 2,989,438 und US 3,817,831 bekannt. Verfahren die in in der Kombination von Autolyse und enzymatischer Proteolyse bestehen, sind aus der US 2,884,385 und US 3,016,331 bekannt. Infolge der für die spezifische Menge der Organe erforderlichen proteolytischen Enzyme ist die Wirtschaftlichkeit dieser Verfahren noch nicht zufriedenstellend.

Die Extraktion erfordert wegen der Proteolyse und der im mehreren Stufen durchgeführten physikalisch/chemischen Maßnahmen viel Zeit und ist mit hohem Aufwand verbunden, weil wegen der sehr geringen Wirkstoffkonzentrationen Vorrichtungen mit großen Volumina angelegt und betrieben werden müssen. Bei der Extraktion mit Chemikalien oder proteolytischen Enzymen fallen die Kosten der eingesetzten Stoffe ins Gewicht. Die Reproduktion ist unsicher, da infolge der verschiedenen Sammel- und Lagerungsbedingungen der Wirkstoffgehalt innerhalb weiter Grenzen schwankt. Nachteilig ist ferner, dass die Aufarbeitbarkeit der in größeren Mengen vorhandenen Verunreinigungen ungünstig beeinflusst wird.

Die bei der Haprinherstellung üblichen Organsammelverfahren ermöglichen es demnach nicht, den nach der Lagerung noch verbliebenen Heparingehalt ohne nennenswerten Verlust in Form von reinem Heparin zu gewinnen. Die Extraktionsverfahren mussten der Qualität des Ausgangsmaterials angepasst werden. Dessen schwankende Qualität erfordert es, in den Extraktionsvorgang Arbeitsgänge, welche die Selektivität vermindern, die Kompliziertheit jedoch erhöhen, einzuschalten.

Aus dem Stand der Technik sind weiterhin Verfahren zur chemischen und enzymatischen Degradation von Heparin bekannt, z.B. aus DE 3102621 C2, DE 4217916 C2, EP 116801 B1, EP 121067 B1, EP 244235 B1, EP 337327 A1, EP 557887 B1, EP 076279 B1, US 6326476 und EP 244236 A2.

Zur Verbesserung und der antitrombotischen Leistungen wurden Versuche angestellt, den allein wirksamen Anteil des Heparinmoleküls zu synthetisieren. Die betreffende chemische Synthese ist beispielsweise in Mauriz Petitou und Constant A.A. Boeckel, Angewandte Chemie 2004, 116, 3180-3196, Weili-VCH-Verlag beschrieben.

Aus Furth, J., Hagen P. und Hirsch, E.I., Proc. Soc. Exp. Biol. Med. 95, 824-828, 1957 ist die Möglichkeit der In-vivo-Produktion von Heparin in wuchendern Mastzellen beschrieben.

Aus Rebecca 1. Montgomery u.a., Proc. Natl. Acad. Sci. USA, Vol. 89, 11327-11331, 1992 ist die In-vitro-Gewinnung von Heparin in stabilen Zelllinien, die aus Mäusen isoliert wurden, bekannt.

Schließlich ist in der WO03/035886 A2 ein Verfahren zur Herstellung von Heparin mit SV-40 transfizierten und nicht-transfizierten porzinen Zellkulturen bekannt. Die Kultivierung der Zellen erfolgt entweder in Suspensionskulturen bis zu 2 Litern oder in Zellkulturflaschen.

Die vorliegende Erfindung hat sich nunmehr die Aufgabe gestellt, die beschriebenen Nachteile des Standes der Technik zu lösen. Insbesondere soll der Aufwand für Lösungsmittel und Chemikalien für die Extraktion verbessert werden. Ferner sollen Produktion und Aufarbeitung technisch und wirtschaftlich vorteilhafter gestaltet werden. Das Verfahren soll sowohl für den Batch-Betrieb als auch den kontinuierlichen Betrieb einsetzbar sein.

Gegenstand der Erfindung ist mithin ein Verfahren zur biotechnologischen Herstellung von sulfatierten Polysacchariden, insbesondere von Heparin mittels tierischer Zellen.

Das Verfahren zeichnet sich dadurch aus, dass die Zellen in immobilisierter Form eingesetzt werden.

Die Immobilisierung der Zellen erfolgt an geeigneten Trägermaterialien. Erfindungsgemäß bevorzugt ist der Einschluss in semipermeable Mikrokapseln. Ebenso kann die Immobilisierung auch an verschiedenen üblichen Biopolymeren vorgenommen werden.

Geeignete Verfahren zur Herstellung von Mikrokapseln sind beispielsweise aus der DE 19756499 C2 bekannt. Beispielsweise kann danach eine Mikrokapsel mit einer Membranhülle aus Polyelektrolyten hergestellt werden. Geeignete Polyelektrolytkomplexe enthalten z.B. Natriumzellulosesulfat (NaCS) und Polydiallydimethlyamoniumchlorid (PDAMAC) oder Natriumzellulosesulfat (NaCS) und Poly-L-Carnitinallylester (PLCA).

Zur Herstellung der mit Zellsubstanz gefüllten Mikrokapseln werden vorzugsweise Kombinationen von anionischen Polymerlösungen und kationischen Polymerlösungen eingesetzt. Als anionische Polymerlösung kommt insbesondere Natriumzellulosesulfat (NaCS) in Betracht

Als kationische Polymerlösungen kommen insbesondere in Betracht Polydiallydimethlyamoniumchlorid (PDAMAC) oder Poly-L-Carnitinallylester (PLCA). PLCA enthält im Gegensatz zu PDADMAC keine tertiären Amine, die gegen Zellen, insbesondere gegen tierische Zellen, toxisch wirken.

Eine erfindungsgemäß besonders bevorzugte Polymerkombination enthält NaCS mit einem Polymerisationsgrad von DP ≥ 2300 und Poly-L-Carnitinallylester (PLCA) mit einem Polymerisationsgrad ≥ 25. Diese Polymerkombination ist für einen besonders schonende Kultivierung der empfindlichen Zellen in Kapseln geeignet, da PLCA Hydroxylgruppen enthält. Diese verhindern ein tiefes Eindringen des Polymers in die Membranstruktur der Kapseln, weil die Hydroxlgruppen Wasserstoffbrückenbindungen mit den Hydroxlgruppen von NaCS ausbilden. Dadurch wird der Kontakt von Polykation mit den Zellen erschwert. Des Weiteren tragen die Wasserstoffbrückenbindungen zur Stabilisierung der Membran bei. Die Kapseln aus den o. g. Polymeren erreichen Stabilitäten von ≥ 3 Newton.

Bei dem Verfahren der Herstellung wird eine Zellsuspension von einer anionischen Polymerlösung umschlossen. D.h., ein mit Zellsuspension gefüllter Tropfen einer anionischen Polymerlösung wird in ein Fällbad gegeben. Dieses Fällbad enthält eine kationische Polymerlösung, z. B. Polydiallydimethlyamoniumchlorid (PDAMAC) oder Poly-L-Carnitinallylester (PLCA).

Nach dem Aushärten wird eine Mikrohohlkugel enthaltend eine Zellsuspension dem weiteren Verfahren zugeführt. Diese Mikrohohlkugel besteht aus zwei Membranen, einer Primärmembran und einer Sekundärmembran. Die Primärmembran hat eine Dicke von 1 bis 5 µm, vorzugsweise 2 bis 3 µm und die Sekundärmembran eine Dicke von 10 bis 150 µm, vorzugsweise 30 bis 70 µm.

Die in der Hohlkugel vorhandene Zellsuspension stammt aus tierischen Geweben, z. B. aus der Darmschleimhaut, der Lunge oder der Leber. Erfindungsgemäß bevorzugt ist der Einsatz von Mastzellen.

Die beschriebene Einschlussimmobilisierung bewirkt während des folgenden Fermentationsprozesses einen Schutz der Zellen von Wirbel- und Strömungskräften in den bewegten Zellkulturmedien. Es werden Zellkonzentrationen von 5x10⁵ bis 1x10⁸, bevorzugt 1x10⁶ pro ml ermöglicht. Die Kulturführung kann entweder als Batch- oder Repeated-Batch-Prozess oder als kontinuierliches Verfahren durchgeführt werden.

Der Vorteil des erfindungsgemäßen Verfahrens liegt darin, dass das Produkt konzentriert in den Kapseln vorliegt. Die Produktkonzentration liegt bei 0,5 bis 1,5 mg/ml, vorzugsweise 1 bis 1,5 mg/ml. Außerdem ist die Aufarbeitung hierdurch vereinfacht, weil nach Gewinnung der Mikrokapseln aus der Kultursuspension aus den Kapseln direkt das Produkt gewonnen werden kann. Das erhaltene Produkt ist nahezu pyrogenfrei.

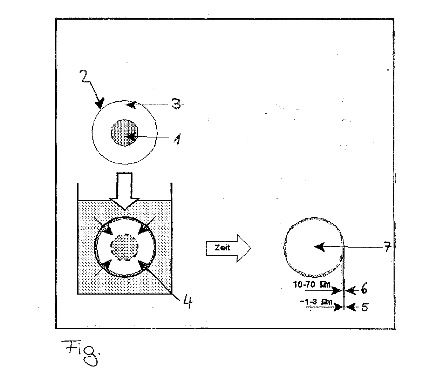

Im folgenden wird die Erfindung unter Bezugnahme auf die Figur näher beschrieben:
In der Figur ist der mit Zellsuspension 1 gefüllte Tropfen 2 mit der die Zellsuspension 1 umfassenden anionischen Polymerlösung 3 zu erkennen. Die Tropfen wird in das Fällbad 4 gegeben. Nach durchgeführter Reaktion kann dem Fellbad die ausgehärtete Mikrohohlkugel 7 entnommen werden, welche die Primärmembran 5 und die Sekundärmembran 6 aufweist. Die erhaltenen Mikrohohlkugeln können anschließend in einem Rührfermenter der weiteren Kultivierung zugeführt werden.

## Patentansprüche

1. Verfahren zur biotechnologischen Herstellung von sulfatierten Polysacchariden mittels tierischen Zellen,
**dadurch gekennzeichnet, dass** die Zellen in immobilisierter Form eingesetzt werden.

2. Verfahren nach Anspruch 1,
**dadurch gekennzeichnet, dass** die Immobilisierung in semipermeablen Mikrokapseln erfolgt.

3. Verfahren nach Anspruch 1,
**dadurch gekennzeichnet, dass** Mikrokapseln eingesetzt werden, die Membranen aus Polyelektrolyten aufweisen.

4. Verfahren nach Anspruch 1,
**dadurch gekennzeichnet, dass** Mikrokapseln eingesetzt werden, die eine Primär- und eine Sekundärmembran aufweisen.

5. Verfahren nach einem der vorhergehenden Ansprüche,
**dadurch gekennzeichnet, dass** Mikrokapseln eingesetzt werden, deren Membran Natriumzellulosesulfat und Polydiallyldimethylamonium-chlorid aufweisen.

6. Verfahren nach einem der vorhergehenden Ansprüche,
**dadurch gekennzeichnet, dass** Mikrokapseln eingesetzt werden, deren Membran Natriumzellulosesulfat und Poly-L-Carnitinallylester aufweisen.

7. Verfahren nach einem der vorhergehenden Ansprüche,
**dadurch gekennzeichnet, dass** Mikrokapseln eingesetzt werden, bei denen die Primärmembran eine Dicke von 1 bis 5 µm und die Sekundärmembran eine Dicke von 10 bis 150 µm aufweisen.

8. Verfahren nach Anspruch 6,
**dadurch gekennzeichnet, dass** die Dicke der Primärmembran 2 bis 3 µm und die Dicke der Sekundärmembran 30 bis 70 µm betragen

9. Verfahren nach einem der vorhergehenden Ansprüche,
**dadurch gekennzeichnet, dass** Mikrokapseln eingesetzt werden, welche Mastzellen enthalten.

10. Verfahren nach einem der vorhergehenden Ansprüche,
**dadurch gekennzeichnet, dass** Mikrokapseln eingesetzt werden, bei denen die Zellkonzentrationen von 5x10⁵ bis 1x10⁸ pro ml liegen.

11. Verfahren nach einem der vorhergehenden Ansprüche,
**dadurch gekennzeichnet, dass** Mikrokapseln eingesetzt werden, die tierische Zellen in Konzentrationen von 1x10⁷ bis 1x10⁸ pro ml enthalten.

12. Verfahren nach einem der vorhergehenden Ansprüche,
**dadurch gekennzeichnet, dass** die Mikrokapseln in einen Rührfermenter gegeben werden.

13. Verfahren nach einem der vorhergehenden Ansprüche,
**dadurch gekennzeichnet, dass** die Fermentation im Batch-, Repeated- Batch-Betrieb oder kontinuierlichen Verfahren durchgeführt wird.

14. Verfahren nach einem der vorhergehenden Ansprüche,
**dadurch gekennzeichnet, dass** die in den Kapseln angereicherten Produkte aufbereitet werden.

15. Verfahren nach einem der vorhergehenden Ansprüche,
**dadurch gekennzeichnet, dass** die Produktkonzentration in den Mikrokapseln bei 0,5 µm bis 1,5 µm pro ml liegt

16. Verfahren nach einem der vorhergehenden Ansprüche,
**dadurch gekennzeichnet, dass** die Konzentration des erzeugten Produkts in den Mikrokapseln bei 1 mg/ml bis 1,5 mg/ml liegt.

17. Verfahren nach einem der vorhergehenden Ansprüche,
**dadurch gekennzeichnet, dass** Heparin hergestellt wird.
